# EUROPÄISCHE PATENTSCHRIFT

(11) **EP 3 032 999 B1**
(45) Veröffentlichungstag und Bekanntmachung des Hinweises auf die Patenterteilung: **25.09.2019**
(21) Anmeldenummer: 14750238.9
(22) Anmeldetag: 12.08.2014
(51) Int. Cl.: A61B 5/00, A61B 5/0408

(54) **TEXTILE KAPAZITIVE ELEKTRODE UND VERFAHREN ZU DEREN HERSTELLUNG**
CAPACITIVE TEXTILE ELECTROD AND METHOD FOR PRODUCING IT
ÉLECTRODE CAPACITIVE TEXTILE ET PROCÉDÉ DE FABRICATION DE LADITE ÉLECTRODE

(30) Priorität: 14.08.2013 DE 102013108810
(43) Veröffentlichungstag der Anmeldung: 22.06.2016
(73) Patentinhaber: Capical GmbH, 38106 Braunschweig (DE)
(72) Erfinder: OEHLER, Martin, 38108 Braunschweig (DE); GRONOSTAY, Heiko, 38124 Braunschweig (DE); BÖGE, Henning, 38106 Braunschweig (DE)
(74) Vertreter: Günther, Constantin
(86) Internationale Anmeldenummer: PCT/EP2014/067249
(87) Internationale Veröffentlichungsnummer: WO 2015/022327

(56) Entgegenhaltungen:
- EP-A1- 2 000 355
- WO-A2-2012/149466
- DE-A1-102006 038 362
- DE-A1-102008 049 112
- US-A- 5 010 772

## Beschreibung

Die Erfindung betrifft eine textile kapazitive Elektrode zur kapazitiven Messung von elektrischen Signalen, insbesondere von Biosignalen, gemäß dem Oberbegriff des Anspruchs 1. Die Erfindung betrifft ferner ein Verfahren zur Herstellung einer textilen kapazitiven Elektrode gemäß Anspruch 8.

Allgemein betrifft die Erfindung das Gebiet kapazitiver Elektroden zur kapazitiven Messung von elektrischen Signalen. Mittels des kapazitiven Erfassungsprinzips können elektrische Signale ohne direkte galvanische Kontaktierung mit einem zu erfassenden Körper erfasst werden. Solche kapazitiven Elektroden eignen sich z. B. für die Erfassung von Biosignalen an Lebewesen, z. B. für die Erfassung von EKG-Signalen oder Herzfrequenz-Signalen.

Ein Vorschlag für eine textile kapazitive Elektrode geht bereits aus der DE 10 2008 049 112 A1 hervor. Aus der US 5,010,772 geht eine kapazitive Druckmessmatrix hervor. Aus der WO 2012/149466 A2 geht eine körperbezogene Einrichtung und ein Verfahren zur Herstellung der Einrichtung hervor.

Dem gegenüber besteht die Aufgabe der vorliegenden Erfindung darin, eine textile kapazitive Elektrode anzugeben, die einfacher und rationeller gefertigt werden kann und sich damit besonders für die Großserienproduktion eignet. Ferner soll ein vorteilhaftes Herstellverfahren für eine solche Elektrode angegeben werden.

Diese Aufgabe wird durch eine Elektrode gemäß Anspruch 1 gelöst.

Die Erfindung hat den Vorteil, dass die genannte textile kapazitive Elektrode besonders effizient mit gängigen Maschinen der industriellen Produktion automatisiert hergestellt werden kann. Notwendige manuelle Tätigkeiten werden eliminiert bzw. auf ein Minimum reduziert. Bei bisherigen Vorschlägen war es z. B. erforderlich, zur Herstellung einer Elektrodenfläche, d. h. einer elektrisch leitfähigen Schicht aus einem Textilmaterial ein leitfähiges Garn in das Textilmaterial einzuweben oder einzusticken. Im Unterschied hierzu wird gemäß einem Aspekt der vorliegenden Erfindung vorgeschlagen, wenigstens eine, mehrere oder alle der elektrisch leitfähigen Schichten zumindest teilweise aus vorgefertigtem textilen EMV-Abschirmmaterial zu bilden. Dies hat große herstellungstechnische Vorteile. Elektrisch leitfähige textile Flächenware ist als Meterware erhältlich. Die elektrisch leitfähige textile Flächenware kann insbesondere als textiles EMV-Abschirmmaterial ausgebildet sein. Die Abkürzung EMV steht für elektromagnetische Verträglichkeit. EMV-Abschirmmaterialien werden ansonsten z.B. für die Abschirmung von Räumen in Gebäuden gegen elektromagnetische Einstrahlung verwendet. EMV-Abschirmmaterialien in textiler Form, bzw. allgemein elektrisch leitfähige textile Flächenwaren sind als fertige Produkte auf dem Markt erhältlich, z. B. von der Firma Statex oder von der Firma Aaronia, z. B. das Abschirmvlies X-Dream. Solche Produkte sind als Meterware auf der Rolle erhältlich und können dementsprechend einfach automatisiert verarbeitet werden.

Bei bisherigen Vorschlägen war es erforderlich, die einzelnen Schichten des mehrschichtigen Aufbaus der Elektrode durch Vernähen mechanisch zusammenzufügen. Solche Nähvorgänge sind mit vergleichsweise hohem Zeitaufwand verbunden, selbst wenn automatische Nähmaschinen oder Nähroboter verwendet werden. Zudem ist ein solcher Herstellprozess auf Grund filigraner Fadenführungen nicht so stabil wie gewünscht. Es kann zu Ausfall- und Wartungszeiten kommen. Im Unterschied hierzu wird gemäß einer vorteilhaften Weiterbildung der vorliegenden Erfindung vorgeschlagen, die wenigstens zwei elektrisch leitfähigen Schichten durch Verkleben miteinander und/oder mit der Isolationsschicht mechanisch zu verbinden. Dies kann ebenfalls sehr rationell automatisiert werden. Die Klebeverbindung ist dann anhand des verwendeten Klebstoffs charakterisiert.

Bei bekannten Elektroden bestand zudem das Problem des Bereitstellens passender Textilstücke, z. B. für die elektrisch leitfähigen Schichten. Ein Zuschneiden von Hand ist für eine Großserienproduktion relativ unwirtschaftlich. Die Verwendung von Schneidrobotern, z. B. in Form von Schneidplottern, führt nicht immer zu der gewünschten Schnittqualität an den Rändern der ausgeschnittenen Stücke, die ausfransen können. Zudem unterliegen die verwendeten Schneidmesser je nach verwendetem Textilmaterial einem relativ großen Verschleiß, so dass Zeit und Kosten für die Wartung solcher Anlagen wiederum die Fertigung weniger wirtschaftlich machen. Die verwendeten Textilmaterialien sind zum Teil auch schlecht mechanisch schneidbar. Im Unterschied hierzu wird gemäß einem Aspekt der vorliegenden Erfindung vorgeschlagen, wenigstens eine, mehrere oder alle der elektrisch leitfähigen Schichten als Laserstrahl-berandete Textilstücke auszubilden. Die Textilstücke können damit direkt über einen Laserstrahl aus dem Rohmaterial, z. B. der vorgefertigten elektrisch leitfähigen textilen Flächenware und/oder dem vorgefertigten textilen EMV-Abschirmmaterial, herausgeschnitten werden. Das Laserschneiden, z.B. mittels einer computergesteuerten Laserstrahl-Schneideinrichtung, erlaubt eine schnelle Bereitstellung der Textilstücke in der gewünschten Form, wobei die Form auch beliebig unregelmäßig geschnitten werden kann. Zudem sind die bereitgestellten Textilstücke besser weiterverarbeitbar und fransen nicht so leicht aus.

Gemäß der Erfindung ist wenigstens eine Verstärkerelektronik zur Verstärkung der von der kapazitiven Elektrode abgegebenen elektrischen Signale in den mehrschichtigen Aufbau der kapazitiven Elektrode integriert. Dies hat den Vorteil, dass die von der kapazitiven Elektrode bzw. von dessen elektrisch leitfähigen Schichten bereitgestellten elektrischen Größen direkt vor Ort elektrisch weiterverarbeitet werden können und dabei insbesondere verstärkt werden können. Dies kommt der Signalqualität zugute. Bei einer weiter entfernt angeordneten Verstärkerelektronik bestünde das Risiko von Einstreuungen in die Zuleitungen. Dies kann bei der erfindungsgemäßen Elektrode vermieden werden. Es kann eine kompakte kapazitive Elektrode bereitgestellt werden, die an ihren äußeren elektrischen Anschlüssen bereits durch die Verstärkerelektronik aufbereitete elektrische Signale bereitstellt und die damit direkt an weiterverarbeitende elektrische Einrichtungen angeschlossen werden kann. Die Verstärkerelektronik kann einen beliebigen, für die jeweilige Anwendung geeigneten Verstärkungsfaktor haben. Der Verstärkungsfaktor kann kleiner oder größer als 1 oder auch gleich 1 sein.

Generell hat die textile kapazitive Elektrode den Vorteil, dass sie insgesamt flexibel ausgebildet werden kann. Dies erlaubt den Einbau in Gegenständen des Alltags wie Sitzen, Liegen oder in Kleidung. Hierbei kann eine solche flexible Elektrode die auftretenden Bewegungen zumindest zum Teil aufnehmen und verformt sich mit. Durch in solche Gegenstände eingearbeitete Elektroden können z. B. EKG's (Elektrokardiogramme) direkt bei einer auf einem Sitz sitzenden oder auf einer Liege liegenden Person aufgenommen werden.

Die in die textile kapazitive Elektrode integrierte Verstärkerelektronik kann ebenfalls als flexible Verstärkerelektronik ausgebildet sein, z. B. mit einer flexiblen Leiterplatte. Es kann auch eine nicht-flexible Verstärkerelektronik eingesetzt werden. In diesem Fall ist es vorteilhaft, diese flächenmäßig relativ klein zu halten, um die Flexibilität der übrigen Bereiche der textilen kapazitiven Elektrode nicht zu stark zu beeinträchtigen.

Als Textilmaterial sei insbesondere jedes flächenförmige textile Gebilde unabhängig von der Art der Verknüpfung der einzelnen Textilfäden verstanden, wie z.B. Gewebe, Gewirke, Gestricke, Geflechte, Gelege, Nähgewirke, Vliesstoffe und Filze.

Gemäß einer vorteilhaften Weiterbildung der Erfindung ist der Klebstoff zur mechanischen Verbindung der Schichten ein elektrisch leitfähiger Klebstoff, der zugleich auch eine elektrische Verbindung wenigstens einer elektrisch leitfähigen Schicht der Elektrode mit wenigstens einem daran angeschlossenen elektrischen Bauelement herstellt, z. B. mit der Verstärkerelektronik oder mit einem elektrischen Anschlusskabel. Dies hat den Vorteil, dass keine weiteren Maßnahmen für die elektrische Kontaktierung erforderlich sind. Bereits durch den Schritt des Verklebens der einzelnen Schichten miteinander können auch die notwendigen elektrischen Kontaktierungen hergestellt werden. Hierdurch kann die Herstellung der kapazitiven Elektroden in Großserienproduktion weiter signifikant optimiert werden. Bei bekannten Vorschlägen war hingegen eine Kontaktierung z. B. mittels Löt- oder Crimpverbindungen oder mit leitfähigem Garn erforderlich, was den Herstellungsaufwand deutlich erhöht. Zudem erlaubt die elektrische Kontaktierung mittels leitfähigen Klebers eine robustere Ausbildung der textilen kapazitiven Elektrode. Es können keine Löt- oder Crimpverbindungen mehr im Betrieb der Elektrode, z.B. infolge einer Verformung der flexiblen Elektrode, beschädigt werden und brechen. Als Klebstoff kann z. B. ein CW 2400 der Firma CircuitWorks eingesetzt werden. Verwendet werden kann z. B. auch ein zwei Komponenten-Klebstoff mit einem Metallanteil, z. B. einem Silberanteil.

Gemäß einer vorteilhaften Weiterbildung der Erfindung weist die Elektrode wenigstens drei elektrisch leitfähige Schichten aus einem Textilmaterial sowie zwischen den elektrisch leitfähigen Schichten angeordnete Isolationsschichten auf. Bei den wenigstens drei elektrisch leitfähigen Schichten kann insbesondere wenigstens eine Sensorschicht zur kapazitiven Ankopplung des mittels der Elektrode zu messenden Signals, wenigstens eine Guardschicht zur Abschirmung der Sensorschicht gegen äußere Störeinflüsse und wenigstens eine Bezugspotentialschicht vorhanden sein. Die Bezugspotentialschicht ist mit einem Bezugspotential verbunden oder verbindbar, z. B. mit einem Massepotential einer elektrischen Schaltung oder dem Erdpotential. Der Aufbau mit den genannten wenigstens drei elektrisch leitfähigen Schichten hat den Vorteil, dass die erfindungsgemäße Elektrode besonders sensitiv gegenüber den zu erfassenden elektrischen Signalen ist, ohne übermäßig anfällig gegenüber Störsignalen zu sein. Außer den zwei zwischen den drei elektrisch leitfähigen Schichten angeordneten Isolationsschichten kann noch eine dritte Isolationsschicht vorhanden sein, so dass sich insgesamt ein sechsschichtiger Aufbau ergibt. Über die dritte Isolationsschicht kann die Elektrode gegenüber der Umgebung isoliert sein, d. h. die dritte Isolationsschicht ist eine außen liegende Isolationsschicht. Die dritte Isolationsschicht kann insbesondere auf der Bezugspotentialschicht aufgebracht sein.

Gemäß einer vorteilhaften Weiterbildung der Erfindung ist die Guardschicht zwischen der Sensorschicht und der Bezugspotentialschicht angeordnet. Hierdurch wird die Elektrode besonders unempfindlich gegenüber Störeinstrahlungen.

Gemäß einer vorteilhaften Weiterbildung der Erfindung weisen die wenigstens zwei oder wenigstens drei elektrisch leitfähigen Schichten wenigstens eine Sensorschicht zur kapazitiven Einkopplung des mittels der Elektrode zu messenden Signals auf, wobei die Sensorschicht als außen liegende Schicht des mehrschichtigen Aufbaus der Elektrode ausgebildet ist, die an ihrer außen liegenden Seite nicht mit einer Isolationsschicht versehen ist. Hierdurch kann der Aufbau der erfindungsgemäßen Elektrode weiter optimiert werden, sowohl im Hinblick auf einfache und kostengünstige Fertigbarkeit als auch im Hinblick auf die Funktion. Grundsätzlich ist für die Funktion einer kapazitiven Elektrode zwar eine Isolation auf der Außenseite der Sensorschicht erforderlich, bei typischen Einsatzfällen von textilen kapazitiven Elektroden kann diese Isolation aber durch die Anwendungsumgebung bereitgestellt werden, wie z. B. einen Sitzbezug oder die Oberfläche eines Kleidungsstoffs. Dementsprechend kann die erfindungsgemäße Elektrode hinsichtlich ihres Aufbaus und ihrer Herstellung gegenüber bekannten Vorschlägen weiter vereinfacht werden.

Gemäß der Erfindung weist eine Isolationsschicht wenigstens eine mit einer elektrisch leitfähigen Schicht aus Textilmaterial überlappende Aussparung auf, durch die die elektrisch leitfähige Schicht mit einem elektrischen Bauelement elektrisch kontaktiert ist. Dies erlaubt vorteilhaft einen mehrschichtigen Aufbau der Elektrode ohne außen liegende Kontaktleitungen. Die Kontaktierung kann ebenfalls in die Elektrode integriert werden. Das elektrische Bauelement kann z. B. die bereits genannte Verstärkerelektronik oder ein sonstiges Bauteil bzw. eine Anschlussleitung sein. Die genannte Kontaktierung durch eine Aussparung Anschlussleitung sein. Die genannte Kontaktierung durch eine Aussparung in der Isolationsschicht kann bei einer, mehreren oder allen Isolationsschichten der Elektrode realisiert werden.

Die Isolationsschichten können grundsätzlich aus jedem beliebigen Isolationsmaterial hergestellt sein. Es ist vorteilhaft, die Isolationsschichten aus flexiblem Material herzustellen, so dass eine insgesamt flexible textile kapazitive Elektrode geschaffen wird. Gemäß einer vorteilhaften Weiterbildung der Erfindung weisen eine, mehrere oder alle der Isolationsschichten ein isolierendes Textilmaterial auf oder bestehen daraus. Dies hat den Vorteil, dass die Isolationsschichten mit den gleichen Verarbeitungsschritten bereitgestellt werden können wie die elektrisch leitfähigen Schichten, insbesondere mittels Laserzuschnitt durch Zuschneiden von vorgefertigtem Textilmaterial, das z. B. auf einer Rolle oder in einer anderen Darbietungsform bereitgestellt wird.

Die eingangs genannte Aufgabe wird ferner durch ein Verfahren zur Herstellung einer textilen kapazitiven Elektrode gemäß Anspruch 8 gelöst.

Vorteilhaft werden die genannten Schritte a) bis c) in der angegebenen Reihenfolge durchgeführt. Hierdurch kann eine textile kapazitive Elektrode, wie zuvor beschrieben, mit den ebenfalls bereits beschriebenen Vorteilen hergestellt werden.

Das Zuschneiden des elektrisch leitfähigen Textilmaterials und/oder des Isolationsmaterials zu Stücken vorgegebener Größe und Form kann mittels eines Lasers erfolgen. Aus dem Isolationsmaterial werden dann die Isolationsschichten gebildet, aus dem elektrisch leitfähigen Textilmaterial die elektrisch leitfähigen Schichten der Elektrode. Das Isolationsmaterial kann insbesondere ein isolierendes Textilmaterial sein. Das Verkleben der zugeschnittenen Stücke kann zumindest teilweise mittels eines elektrisch leitfähigen Klebstoffs erfolgen. Optional ist zusätzlich ein Vernähen einzelner oder aller Schichten der Elektrode möglich. Es können insgesamt wenigstens drei elektrisch leitfähige Schichten aus einem Textilmaterial vorgesehen sein, zwischen denen jeweils Isolationsschichten angeordnet sind. Die wenigstens drei elektrisch leitfähigen Schichten können wie zuvor für die Elektrode beschrieben ausgebildet sein.

Gemäß der Erfindung wird im Schritt d) des Anspruchs 8 eine Verstärkerelektronik zur Verstärkung der von der kapazitiven Elektrode abgegebenen elektrischen Signale in den mehrschichtigen Aufbau der kapazitiven Elektrode integriert.

Die Erfindung wird nachfolgend anhand von Ausführungsbeispielen unter Verwendung von Zeichnungen näher erläutert. Es zeigen
- Figur 1: - eine Elektrode in einer isometrischen Darstellung nach Art einer Explosionszeichnung und
- Figur 2: - die Elektrode gemäß Figur 1 in einer seitlichen Ansicht und
- Figur 3: - die einzelnen Schichten der Elektrode gemäß Figur 1 in einer Draufsicht und
- Figur 4: - die Elektrode gemäß Figur 1 in einer Draufsicht und
- Figur 5: - die Elektrode gemäß Figur 1 in Draufsicht in einer Art Röntgendarstellung.

In den Figuren werden gleiche Bezugszeichen für einander entsprechende Elemente verwendet.

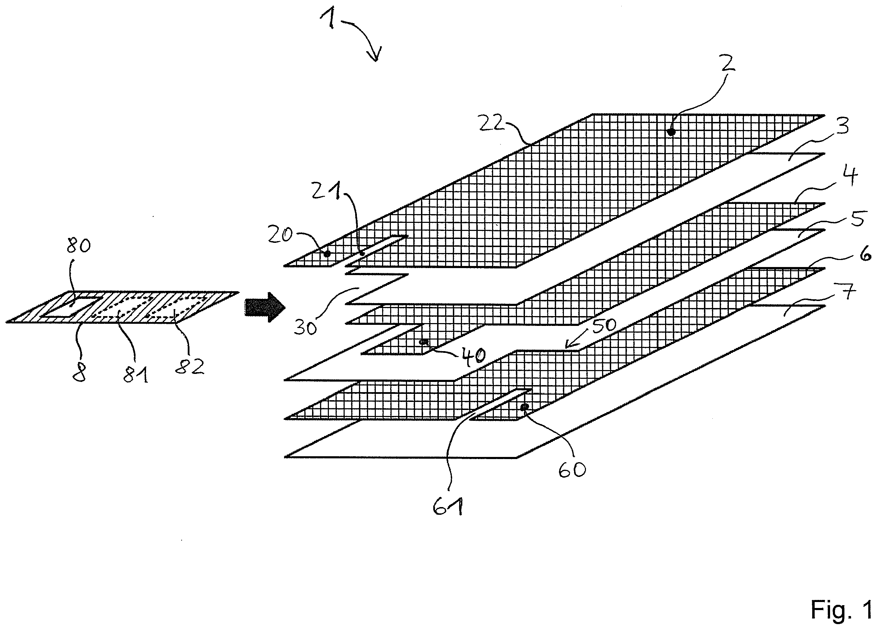

Die Figur 1 zeigt die Elektrode 1 mit den einzelnen Schichten in einer isometrischen Ansicht, bevor die Schichten zusammengeklebt sind. Erkennbar sind drei elektrisch leitfähige Schichten 2, 4, 6 aus einem elektrisch leitfähigen Textilmaterial sowie drei Isolationsschichten 3, 5, 7 aus einem isolierenden Textilmaterial. Die oberste elektrisch leitfähige Schicht 2 ist die Sensorschicht der Elektrode, die zur kapazitiven Einkopplung des mittels der Elektrode zu messenden Signals dient. Die mittlere elektrisch leitfähige Schicht 4 ist eine Guardschicht, die zur Abschirmung der Sensorschicht 2 gegen äußere Störeinflüsse, insbesondere ESD-Einflüsse, dient. Die untere elektrisch leitfähige Schicht 6 ist eine Bezugspotentialschicht, die mit einem Bezugspotential zu verbinden ist. Die Sensorschicht 2 weist an einer Ecke eine Aussparung 21 auf, durch die eine Kontaktlasche 20 zur elektrischen Kontaktierung der Sensorschicht 2 gebildet wird. Die Guardschicht 4 weist eine Kontaktlasche 40 auf, die dadurch gebildet ist, dass links und rechts von der Kontaktlasche 40 Stücke vom Textilmaterial der Guardschicht 4 abgetrennt worden sind. Die Kontaktlasche 40 dient zur elektrischen Kontaktierung der Guardschicht 4. Die Bezugspotentialschicht 6 ist vergleichbar ausgebildet wie die Sensorschicht 2, jedoch mit einer Kontaktlasche 60 auf der gegenüberliegenden Seite. Die Kontaktlasche 60 wird in Folge einer Aussparung 61, die aus dem Textilmaterial der Bezugspotentialschicht 6 herausgeschnitten ist, gebildet. Die oberste Isolationsschicht 3 weist an einer Ecke eine Aussparung 30 auf, die unterhalb der Kontaktlasche 20 liegt. Die mittlere Isolationsschicht 5 weist an einer gegenüberliegenden Ecke derselben Seite eine Aussparung 50 auf. Die Aussparung 50 überlappt mit der Kontaktlasche 60. Die unterste Isolationsschicht 7 weist keine solchen Aussparungen auf. Die Schichten 2, 3, 4, 5, 6, 7 können z. B. durch Laserschneiden in die beschriebene und dargestellte äußere Kontur gebracht werden. Mit einem Laser werden die äußeren Konturen abgefahren, wodurch auch leicht die genannten Aussparungen erzeugt werden können. Es entsteht dadurch eine Laserstrahl-Berandung der Textilstücke, wie sie beispielhaft in Form einer Berandung 22 der Sensorschicht 2 dargestellt ist.

Die äußere Form der Elektrode 1 bzw. der einzelnen Schichten 2, 3, 4, 5, 6, 7 muss nicht unbedingt, wie in Figur 1 dargestellt, im Wesentlichen rechteckig sein, sondern kann jede andere gewünschte Form einnehmen, wie z. B. oval, rechteckig mit abgerundeten Ecken oder kreisrund.

In dem in Figur 1 dargestellten mehrschichtigen Aufbau wird in dem Bereich, in dem die Kontaktlaschen 20, 40, 60 vorhanden sind, eine Verstärkerelektronik 8, die zur Verstärkung der von der kapazitiven Elektrode 1 abgegebenen elektrischen Signale dient, integriert. Die Verstärkerelektronik 8 wird dabei zwischen der oberen Isolationsschicht 3 und der Guardschicht 4 angeordnet. Dies ist jedoch nur ein Beispiel für eine mögliche Anordnung, auch andere Positionierungen der Verstärkerelektronik 8 sind vorteilhaft möglich. Die Verstärkerelektronik 8 weist an der in Figur 1 oben dargestellten Seite eine elektrische Anschlussfläche 80 auf, und an der unteren Seite zwei weitere elektrische Anschlussflächen 81, 82, z. B. in Form von Kontaktpads. In Folge der Aussparungen 30, 50 kann die Kontaktlasche 20 elektrisch mit der Anschlussfläche 80 verbunden werden, die Kontaktlasche 40 mit der Anschlussfläche 81 und die Kontaktlasche 60 mit der Anschlussfläche 82. In Folge der in den übrigen Bereichen überlappenden Isolationsschichten 3, 5, 7 kann es nicht zu ungewollten Kurzschlüssen oder Fehlkontaktierungen kommen.

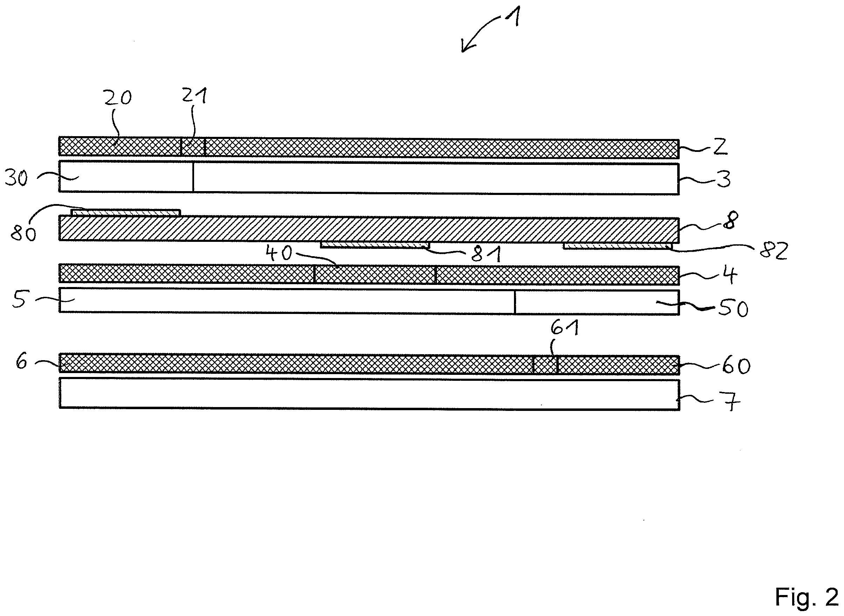

Die Figur 2 zeigt die Elektrode 1 gemäß Figur 1 in einer Seitenansicht mit Blick auf diejenige Schmalseite der Elektrode 1, an der die Verstärkerelektronik 8 eingesetzt ist. Erkennbar sind die auf unterschiedlichen Seiten der Verstärkerelektronik 8 angeordneten Anschlussflächen 80, 81, 82 sowie deren Kontaktierungsmöglichkeit durch die Isolationsschichten 3, 5 hindurch zu den Kontaktlaschen 20, 40, 60.

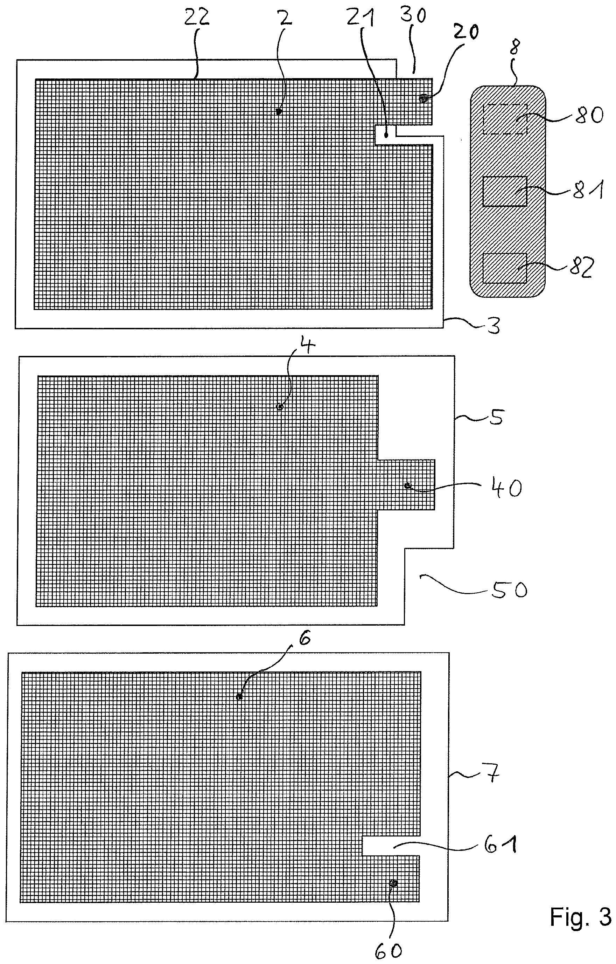

Die Figur 3 zeigt die einzelnen Schichten 2, 3, 4, 5, 6 sowie die Verstärkerelektronik 8 noch einmal teilweise nebeneinander in einer Draufsicht, wobei jeweils eine elektrisch leitfähige Schicht 2, 4, 6 oberhalb von der ihr zugeordneten, darunterliegenden Isolationsschicht 3, 5, 7 dargestellt ist.

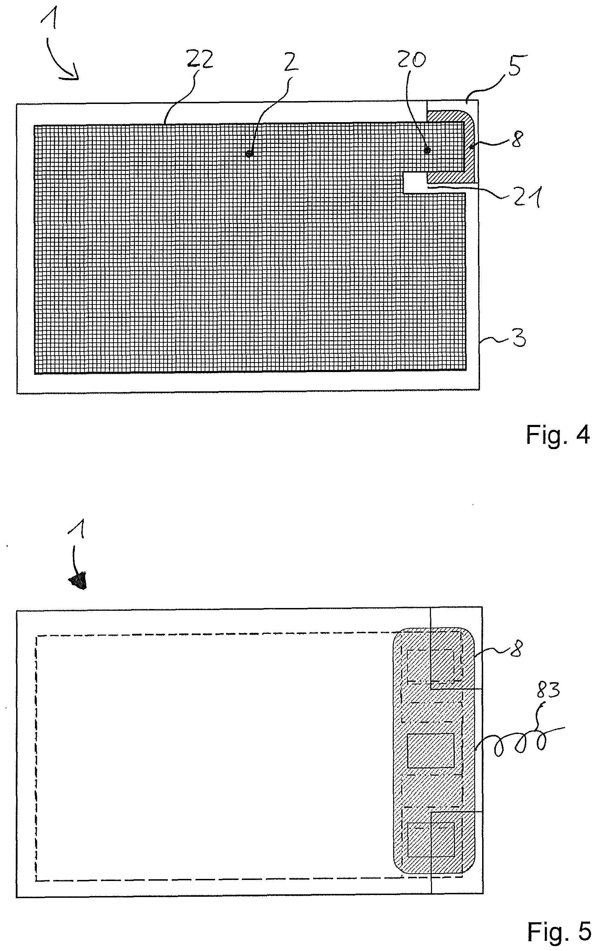

Die Figur 4 zeigt die in Figur 3 dargestellten Elemente nach ihrem Zusammenbau zur Elektrode 1, d. h. nachdem die einzelnen Schichten miteinander mit einem elektrisch leitfähigen Kleber verklebt wurden. Die Figur 5 zeigt die Elektrode 1 gemäß Figur 4 in einer Art Röntgendarstellung, in der auch tiefer liegende Schichten sichtbar gemacht sind. Erkennbar sind insbesondere die Konturen der Verstärkerelektronik 8, der Anschlussflächen 80, 81, 82 sowie der verschiedenen Kontaktlaschen 20, 40, 60 und Aussparungen 30, 50.

Die Figur 5 zeigt beispielhaft noch die elektrische Kontaktierung der Elektrode 1 mittels einer elektrischen Anschlussleitung 83. Mittels der elektrischen Anschlussleitung 83 können die von der Verstärkerelektronik 8 abgegebenen elektrischen Signale einer Nutzanwendung zugeführt werden, z. B. einem EKG-oder Herzfrequenz-Erfassungssystem.

Die beschriebene Elektrode eignet sich für eine Integration in Sitze, insbesondere Sitzmöbel oder Fahrzeugsitze, in Liegen, z. B. zur Patientenüberwachung, sowie zur Kleidungsintegration. Wichtige Anwendungsgebiete der Elektrode sind die EKG- und Herzfrequenzmessung.

Wie erkennbar ist, kann die Herstellung der erfindungsgemäßen Elektrode für eine automatisierte Fertigung stark optimiert werden. Es können z. B. folgende Schritte durchgeführt werden:
- Verarbeitung von elektrisch leitfähiger und isolierender textiler Meterware,
- Zuschnitt der Textilien per Laser, z. B. von einer Vorratsrolle oder aus Zuschnitten des Textilmaterials,
- Form des Zuschnitts so durchführen, dass eine spätere Verklebung und Isolierung bzw. elektrische Kontaktierung bereits durch das Design des Zuschnitts erleichtert wird,
- Verklebung der einzelnen Textilschichten miteinander, z.B. durch pressen des mehrschichtigen Aufbaus,
- Nutzung von elektrisch leitfähigem Kleber zur gleichzeitigen elektrischen Kontaktierung,
- Verklebung einer Verstärkerelektronik in den mehrschichtigen Aufbau,
- ggf. optional ein Vernähen der Schichten.

## Patentansprüche

1. Textile kapazitive Elektrode (1) zur kapazitiven Messung von elektrischen Signalen, insbesondere von Biosignalen, wobei die Elektrode (1) einen mehrschichtigen Aufbau aufweist, der wenigstens zwei elektrisch leitfähige Schichten (2, 4, 6) aus einem Textilmaterial und wenigstens eine zwischen den wenigstens zwei elektrisch leitfähigen Schichten (2, 4, 6) angeordnete Isolationsschicht (3, 5, 7) und wenigstens eine Verstärkerelektronik (8) zur Verstärkung der von der kapazitiven Elektrode (1) abgegebenen elektrischen Signale aufweist, wobei die Verstärkerelektronik (8) in den mehrschichtigen Aufbau der kapazitiven Elektrode (1) integriert ist und mindestens zwei Anschlussflächen (80, 81, 82) aufweist, wobei die wenigstens zwei elektrisch leitfähigen Schichten (2, 4, 6) eines oder alle der folgenden Merkmale a) oder b) aufweisen:
a) wenigstens eine, mehrere oder alle der elektrisch leitfähigen Schichten (2, 4, 6) weisen ein vorgefertigtes textiles EMV-Abschirmmaterial auf oder bestehen daraus,
b) wenigstens eine, mehrere oder alle der elektrisch leitfähigen Schichten (2, 4, 6) sind als Laserstrahl-berandete Textilstücke ausgebildet,
wobei die wenigstens eine Isolationsschicht (3, 5, 7) wenigstens eine Aussparung (30, 50) aufweist, **dadurch gekennzeichnet, dass** jede der wenigstens zwei elektrisch leitfähigen Schichten (2, 4, 6) eine Kontaktlasche (20, 40, 60) aufweist, die aus dem Textilmaterial der elektrisch leitfähigen Schichten (2, 4, 6) herausgeschnitten ist, wobei mindestens eine der Kontaktlaschen (20, 40, 60) die mindestens eine Aussparung (30, 50) überlappt und durch die mindestens eine Aussparung mit einem der mindestens zwei Anschlussflächen (80, 81, 82) elektrisch kontaktiert ist.

2. Elektrode nach Anspruch 1, **dadurch gekennzeichnet, dass** die wenigstens zwei elektrisch leitfähigen Schichten (2, 4, 6) durch Verkleben miteinander und/oder mit der Isolationsschicht (3, 5, 7) mechanisch verbunden sind und der Klebstoff zur mechanischen Verbindung der Schichten (2, 3, 4, 5, 6, 7) ein elektrisch leitfähiger Klebstoff ist, der zugleich auch eine elektrische Verbindung wenigstens einer elektrisch leitfähigen Schicht (2, 4, 6) mit wenigstens einem daran angeschlossenen elektrischen Bauelement herstellt.

3. Elektrode (1) nach einem der vorhergehenden Ansprüche, **dadurch gekennzeichnet, dass** die Elektrode wenigstens drei elektrisch leitfähige Schichten (2, 4, 6) aus einem Textilmaterial sowie zwischen den elektrisch leitfähigen Schichten (2, 4, 6) angeordnete Isolationsschichten (3, 5, 7) aufweist.

4. Elektrode nach Anspruch 3, **dadurch gekennzeichnet, dass** die wenigstens drei elektrisch leitfähigen Schichten (2, 4, 6) wenigstens eine Sensorschicht (2) zur kapazitiven Einkopplung des mittels der Elektrode (1) zu messenden Signals, wenigstens eine Guardschicht (4) zur Abschirmung der Sensorschicht (2) gegen äußere Störeinflüsse und wenigstens eine Bezugspotentialschicht (6) aufweisen, die mit einem Bezugspotential verbunden oder verbindbar ist.

5. Elektrode nach Anspruch 4, **dadurch gekennzeichnet, dass** die Guardschicht (4) zwischen der Sensorschicht (2) und der Bezugspotentialschicht (6) angeordnet ist.

6. Elektrode nach einem der vorhergehenden Ansprüche, **dadurch gekennzeichnet, dass** die wenigstens zwei oder wenigstens drei elektrisch leitfähigen Schichten (2, 4, 6) wenigstens eine Sensorschicht (2) zur kapazitiven Einkopplung des mittels der Elektrode (1) zu messenden Signals aufweisen, wobei die Sensorschicht (2) als außen liegende Schicht des mehrschichtigen Aufbaus der Elektrode (1) ausgebildet ist, die an ihrer außen liegenden Seite nicht mit einer Isolationsschicht (3, 5, 7) versehen ist.

7. Elektrode nach einem der vorhergehenden Ansprüche, **dadurch gekennzeichnet, dass** eine, mehrere oder alle der Isolationsschichten (3, 5, 7) ein isolierendes Textilmaterial aufweisen oder daraus bestehen.

8. Verfahren zur Herstellung einer textilen kapazitiven Elektrode (1) nach einem der vorhergehenden Ansprüche, mit den Schritten:
a) Bereitstellen eines vorgefertigten elektrisch leitfähigen Textilmaterials sowie eines vorgefertigten Isolationsmaterials,
b) Zuschneiden des elektrisch leitfähigen Textilmaterials und des Isolationsmaterials zu Stücken vorgegebener Größe und Form,
c) Verkleben der zugeschnittenen Stücke miteinander zu einer Elektrode (1), die einen mehrschichtigen Aufbau aufweist, der wenigstens zwei elektrisch leitfähige Schichten (2, 4, 6) aus dem Textilmaterial und wenigstens eine zwischen den wenigstens zwei elektrisch leitfähigen Schichten angeordnete Isolationsschicht (3, 5, 7) aufweist,
d) Integrieren wenigstens einer Verstärkerelektronik (8) zur Verstärkung der von der kapazitiven Elektrode (1) abgegebenen elektrischen Signale in den mehrschichtigen Aufbau der kapazitiven Elektrode (1),
**dadurch gekennzeichnet, dass** jede der wenigstens zwei elektrisch leitfähigen Schichten (2, 4, 6) eine Kontaktlasche (20, 40, 60) aufweist, die aus dem Textilmaterial der elektrisch leitfähigen Schichten (2, 4, 6) herausgeschnitten wird, wobei mindestens eine der Kontaktlaschen (20, 40, 60) die mindestens eine Aussparung (30, 50) überlappt und durch die mindestens eine Aussparung mit einem der mindestens zwei Anschlussflächen (80, 81, 82) elektrisch kontaktiert wird.

9. Verfahren nach Anspruch 8, **dadurch gekennzeichnet, dass** Verkleben der zugeschnittenen Stücke miteinander zumindest teilweise mittels eines elektrisch leitfähigen Klebstoffs erfolgt.

## Claims

1. A capacitive textile electrode (1) for the capacitive measurement of electrical signals, in particular of biosignals, wherein the electrode (1) has a multilayer structure, which comprises at least two electrically conductive layers (2, 4, 6) of a textile material and at least one insulating layer (3, 5, 7) arranged between the at least two electrically conductive layers (2, 4, 6) and at least one amplifier electronics system (8) for amplifying the electrical signals emitted by the capacitive electrode (1), wherein the amplifier electronics system (8) is integrated in the multilayer structure of the capacitive electrode (1) and comprises at least two connection areas (80, 81, 82) wherein the at least two electrically conductive layers (2, 4, 6) comprise one or all of the following features a), b) :
a) at least one or more or all of the electrically conductive layers (2, 4, 6) comprise(s) or consist(s) of a prefabricated textile EMC shielding material,
b) at least one or more or all of the electrically conductive layers (2, 4, 6) are formed as textile pieces trimmed by a laser beam,
wherein the at least one insulating layer (3, 5, 7) comprises at least one clearance (30, 50), **characterized in that** each of the at least two electrically conductive layers (2, 4, 6) comprises a contact link (20, 40, 60) which is cut out of the textile material of the electrically conductive layers (2, 4, 6), wherein at least one of the contact links (20, 40, 60) overlaps with the at least one clearance (30, 50) and is electrically connected with one of the at least two contact areas (80, 81, 82) through the at least one clearance.

2. The electrode as claimed in claim 1, **characterized in that** the at least two electrically conductive layers (2, 4, 6) are mechanically connected to one another and/or to the insulating layer (3, 5, 7) by adhesive bonding and the adhesive for the mechanical connection of the layers (2, 3, 4, 5, 6, 7) is an electrically conductive adhesive, which at the same time also establishes an electrical connection between at least one electrically conductive layer (2, 4, 6) and at least one electrical component connected thereto.

3. The electrode (1) as claimed in either of the preceding claims, **characterized in that** the electrode has at least three electrically conductive layers (2, 4, 6) of a textile material and also insulating layers (3, 5, 7) arranged between the electrically conductive layers (2, 4, 6).

4. The electrode as claimed in claim 3, **characterized in that** the at least three electrically conductive layers (2, 4, 6) comprise at least one sensor layer (2) for the capacitive incoupling of the signal to be measured by means of the electrode (1), at least one guard layer (4) for shielding the sensor layer (2) from external interfering influences and at least one reference potential layer (6), which is connected or can be connected to a reference potential.

5. The electrode as claimed in claim 4, **characterized in that** the guard layer (4) is arranged between the sensor layer (2) and the reference potential layer (6) .

6. The electrode as claimed in one of the preceding claims, **characterized in that** the at least two or at least three electrically conductive layers (2, 4, 6) have at least one sensor layer (2) for the capacitive incoupling of the signal to be measured by means of the electrode (1), wherein the sensor layer (2) is formed as an outer layer of the multilayer structure of the electrode (1) that is not provided with an insulating layer (3, 5, 7) on its outer side.

7. The electrode as claimed in one of the preceding claims, **characterized in that** one or more or all of the insulating layers (3, 5, 7) comprise(s) or consist(s) of an insulating textile material.

8. A method for producing a capacitive textile electrode (1) as claimed in one of the preceding claims, with the steps of:
a) providing a prefabricated electrically conductive textile material and also a prefabricated insulating material,
b) cutting to size the electrically conductive textile material and the insulating material into pieces of a predetermined size and form,
c) adhesively bonding the cut-to-size pieces to one another to form an electrode (1) that has a multilayer structure which comprises at least two electrically conductive layers (2, 4, 6) of the textile material and at least one insulating layer (3, 5, 7) arranged between the at least two electrically conductive layers,
d) integrating an amplifier electronics system (8) for amplifying the electrical signals emitted by the capacitive electrode (1) in the multilayer structure of the capacitive electrode (1), **characterized in that** each of the at least two electrically conductive layers (2, 4, 6) comprises a contact link (20, 40, 60), which is cut out of the textile material of the electrically conductive layers (2, 4, 6), wherein at least one of the contact links (20, 40, 60) overlaps with the at least one clearance (30, 50) and is electrically connected through the at least one clearance with one of the at least two contact areas (80, 81, 82).

9. The method as claimed in claim 8, **characterized in that** the adhesive bonding of the cut-to-size pieces to one another takes place at least partially by means of an electrically conductive adhesive.

## Revendications

1. Électrode (1) capacitive textile destinée à la mesure capacitive de signaux électriques, notamment de biosignaux, l'électrode (1) présentant une structure multicouche qui possède au moins deux couches électriquement conductrices (2, 4, 6) en un matériau textile et au moins une couche isolante (3, 5, 7) disposée entre les au moins deux couches électriquement conductrices (2, 4, 6) et au moins une électronique d'amplification (8) destinée à amplifier les signaux électriques délivrés par l'électrode (1) capacitive, l'électronique d'amplification (8) étant intégrée dans la structure multicouche de l'électrode (1) capacitive et possédant au moins deux surfaces de raccordement (80, 81, 82), les au moins deux couches électriquement conductrices (2, 4, 6) possédant une ou la totalité des caractéristiques a) ou b) ci-après :
a) au moins une, plusieurs ou la totalité des couches électriquement conductrices (2, 4, 6) possèdent un matériau de blindage CEM textile préfabriqué ou en sont constituées,
b) au moins une, plusieurs ou la totalité des couches électriquement conductrices (2, 4, 6) sont réalisées sous la forme de pièces textiles détourées au rayon laser, l'au moins une couche isolante (3, 5, 7) possédant au moins un évidement (30, 50), **caractérisée en ce que** chacune des au moins deux couches électriquement conductrices (2, 4, 6) possède une languette de contact (20, 40, 60) qui est découpée à partir du matériau textile des couches électriquement conductrices (2, 4, 6), au moins l'une des languettes de contact (20, 40, 60) chevauchant l'au moins un évidement (30, 50) et étant amenée en contact électrique avec l'une des au moins deux surfaces de raccordement (80, 81, 82) à travers l'au moins un évidement.

2. Électrode selon la revendication 1, **caractérisée en ce que** les au moins deux couches électriquement conductrices (2, 4, 6) sont assemblées mécaniquement par collage les unes avec les autres et/ou avec la couche isolante (3, 5, 7) et l'adhésif servant à l'assemblage mécanique des couches (2, 3, 4, 5, 6, 7) est un adhésif électriquement conducteur qui établit en même temps aussi une liaison électrique entre au moins une couche électriquement conductrice (2, 4, 6) et au moins un composant électrique raccordé à celle-ci.

3. Électrode (1) selon l'une des revendications précédentes, **caractérisée en ce que** l'électrode possède au moins trois couches électriquement conductrices (2, 4, 6) en un matériau textile ainsi que des couches isolantes (3, 5, 7) disposées entre les couches électriquement conductrices (2, 4, 6).

4. Électrode selon la revendication 3, **caractérisée en ce que** les au moins trois couches électriquement conductrices (2, 4, 6) possèdent au moins une couche de détection (2) servant à l'injection par couplage capacitif du signal à mesurer au moyen de l'électrode (1), au moins une couche de barrière (4) destinée à réaliser le blindage de la couche de détection (2) contre les influences parasites externes et au moins une couche de potentiel de référence (6), qui est reliée ou peut être reliée à un potentiel de référence.

5. Électrode selon la revendication 4, **caractérisée en ce que** la couche de barrière (4) est disposée entre la couche de détection (2) et la couche de potentiel de référence (6).

6. Électrode selon l'une des revendications précédentes, **caractérisée en ce que** les au moins deux ou les au moins trois couches électriquement conductrices (2, 4, 6) possèdent au moins une couche de détection (2) servant à l'injection par couplage capacitif du signal à mesurer au moyen de l'électrode (1), la couche de détection (2) étant réalisée sous la forme d'une couche, se trouvant à l'extérieur, de la structure multicouche de l'électrode (1), laquelle n'est pas pourvue d'une couche isolante (3, 5, 7) au niveau de son côté se trouvant à l'extérieur.

7. Électrode selon l'une des revendications précédentes, **caractérisée en ce qu'**une, plusieurs ou la totalité des couches isolantes (3, 5, 7) possèdent un matériau textile isolant ou en sont constituées.

8. Procédé de fabrication d'une électrode (1) capacitive textile selon l'une des revendications précédentes, comprenant les étapes suivantes :
a) fourniture d'un matériau textile électriquement conducteur préfabriqué ainsi que d'un matériau isolant préfabriqué,
b) découpe du matériau textile électriquement conducteur et du matériau isolant en pièces de taille et de forme prédéfinies,
c) collage des pièces découpées entre elles pour obtenir une électrode (1), laquelle présente une structure multicouche qui possède au moins deux couches électriquement conductrices (2, 4, 6) en un matériau textile et au moins une couche isolante (3, 5, 7) disposée entre les au moins deux couches électriquement conductrices,
d) intégration d'au moins une électronique d'amplification (8) destinée à amplifier les signaux électriques délivrés par l'électrode (1) capacitive, dans la structure multicouche de l'électrode (1) capacitive **caractérisé en ce que** chacune des au moins deux couches électriquement conductrices (2, 4, 6) possède une languette de contact (20, 40, 60) qui est découpée à partir du matériau textile des couches électriquement conductrices (2, 4, 6), au moins l'une des languettes de contact (20, 40, 60) chevauchant l'au moins un évidement (30, 50) et étant amenée en contact électrique avec l'une des au moins deux surfaces de raccordement (80, 81, 82) à travers l'au moins un évidement.

9. Procédé selon la revendication 8, **caractérisé en ce que** le collage des pièces découpées entre elles est effectué au moins partiellement au moyen d'un adhésif électriquement conducteur.
